# EUROPEAN PATENT SPECIFICATION

(11) **EP 0 595 188 B1**
(45) Date of publication and mention of the grant of the patent: **30.09.1998**
(21) Application number: 93117015.3
(22) Date of filing: 21.10.1993
(51) Int. Cl.: C07K 2/00, C07K 1/30, C07K 14/285, C07K 14/245, A61K 39/02, A61K 39/102, A61K 39/108

(54) **Process for producing vaccine for a bacterial toxin belonging to the RTX toxin family**
Verfahren zur Herstellung eines Impfstoffes für ein zur RTX-Toxinfamilie gehörendes bakterielles Toxin
Procédé de production d'un vaccin d'une toxine bactérienne appartenant à la famille des toxines RTX

(30) Priority: 27.10.1992 JP 311182/92
(43) Date of publication of application: 04.05.1994
(73) Proprietor: NIPPON ZENYAKU KOGYO CO. LTD., Koriyama City, Fukushima Prefecture (JP)
(72) Inventor: Haga, Yoshihisa, c/o Nippon Zenyku Kogyo Co., Ltd., Koriyama City, Fukushima Prefecture (JP)
(74) Representative: Segeth, Wolfgang

(56) References cited:
- US-A- 4 681 761
- DATABASE WPI Section Ch, Week 8837, Derwent Publications Ltd., London, GB; Class B04, AN 88-262387 & SU-A-1 313 172 (MOSCOW LOMONOSOV UNIV) 15 March 1988

## Description

### BACKGROUND OF THE INVENTION

The present invention relates to a process for producing a vaccine which is effective for prevention of infectious disease induced by RTX-toxin producing bacteria and which is highly safe.

Bacteria such as Actinobacillus pleuropneumoniae, Actinobacillus suis, Pasteurelle haemolytica, Escherichia coli produce a certain exotoxins showing serological cross-reaction to each other. Each of these toxins is a protein having molecular weight of 103 to 110 KDa. Since repeated sequences of a certain amino acid sequence are present in its structural gene, it is called a RTX (repeats in the structural toxin) toxin family. These toxins will cause disorders to cells such as erythrocytes, neutrophiles and macrophages, lyse the cells and finally induce diseases to the host.

For example, A. pleuropneumoniae produces a toxin called hemolysin which belongs to RTX toxin family. This exotoxin is an important pathogen to cause pleuropneumonia in pigs. On the other hand, it has been revealed that a pig immunized with purified hemolysin protein has resistance to subsequent infection of A. pleuropneumoniae (Devenish, J., S. Rosendal and J. T. Bosse, Infect. Immun., 58, 3829 - 3832, 1990) so that hemolysin may be an effective vaccine antigen for prevention of pleuropneumonia in pigs. It was, however, recently found out that hemolysin protein is relatively firmly bonded with lipopolysaccharides derived from cells. Lipopolysaccharide is an endotoxin with very high toxicity and is closely related to production of cytokine which exerts various actions to living body. Therefore, use of hemolysin, which contains lipopolysaccharides, as vaccine antigen for pigs may cause side reactions such as endotoxin shock.

Such characteristics are also found in the other RTX toxins such as α-hemolysin from E. coli (Bohach, G. A., and I. S. Snyder, Infect. Immun., 53, 435 - 437, 1986). This makes it difficult, in terms of safety, to develop prophylactic vaccine to bacterial infectious diseases with which these toxins are involved.

For this reason, a requisite to prepare a safe vaccine for RTX toxin family is to remove from the toxin protein as much lipopolysaccharides as possible which otherwise cause various side reactions.

It is an object of the present invention to efficiently remove lipopolysaccharides from RTX toxin to thereby produce a vaccine for RTX toxin having high safety.

### BRIEF SUMMARY OF THE INVENTION

The present invention is a process for producing a vaccine for bacterial toxin belonging to RTX toxin family which has characteristics as follows; bacteria which produce toxin belonging to RTX toxin family are proliferated in a nutrient enriched medium. A concentrated supernatant of the culture is added with a cationic surface active agent with range of 1 to 10 mM to precipitate RTX toxin, not containing lipopolysaccharides and then the precipitate is washed by distilled water. This precipitate is used as vaccine antigen.

### DETAILED DESCRIPTION OF PREFERRED EMBODIMENTS

To work out the problems in item of above-mentioned background, the inventor studied and researched how to remove lipopolysaccharides from RTX toxin, and found out that cationic surface active agent has an effect of removing lipopolysaccharides.

More specifically, bacteria which produce a toxin belonging to RTX toxin family are proliferated in a nutrient enriched medium and culture supernatant is obtained by centrifugation or the like, then concentrated by ultrafiltration. The concentrated solution is added with a cationic surface active agent which may be lauryltrimethylammonium bromide, tetradecyltrimethylammonium bromide, cetyltrimethyl ammonium bromide, laurylpyridinium chloride, cetylpyridium chloride or the like. Concentration of the surface active agent is preferably 1 to 10 mM. By this procedure, RTX toxin is made insoluble and precipitation occurs, whereby lipopolysaccharides are separated from RTX toxin and remain in the supernatant. The supernatant is removed by centrifugation and 1.0 M salt solution is added to the precipitate to dissolve it. Further, excessive quantity of distilled water is added to precipitate RTX toxin again. This procedure may be repeated, if necessary. These procedures can remove most of the lipopolysaccharides bonded to RTX toxin and purify RTX toxin to higher purity.

The precipitate of RTX toxin such prepared is dissolved in an appropriate solvent and, if necessary, is turned to toxoid. By adding appropriate adjuvant and preservative, a vaccine is prepared.

### Example 1

An examination was carried out to determine required concentration of a cationic surface active agent for removing lipopolysaccharides from RTX toxin. A. pleuropneumoniae HA-337 strain (serotype 1) was used for this study, which was isolated from lungs of a field case of porcine pleuropneumonia in Japan at 1989. Pre-culture of this strain was inoculated to 5 litres of Columbia broth (BBL Microbiology Systems, Cockeysville, Md, USA) supplemented with 0.002% β-NAD and 10 mM calcium chloride and was incubated at 37°C for four hours under aerated agitation. The culture was centrifuged and the supernatant was concentrated to about 500 ml by ultrafiltration (300 KDa cutoff). To 0.5 ml each of the concentrated solution, 0.5 ml each of 0, 2, 4, 10, 20, 40 and 80 mM cetyltrimethylammonium bromide was added and they were sensitized at room temperature for about 30 minutes. Because precipitation occurs during the sensitization, they were separated into supernatant and precipitate by centrifugation. 1.0 ml of 1.0 M sodium chloride was added to the precipitate to dissolve it. Quantities of endotoxin in the precipitate-dissolved solution and the centrifuged supernatant were determined by Limulus test (QCL-1000; Whittaker Bioproducts Inc., Walkersvill, Md, USA). To confirm the presence of hemolysin protein, these were analyzed by sodium dodecyl sulfate-polyacrylamide gel electrophoresis (SDS-PAGE).

Table 1 shows the quantity of endotoxin in the precipitate-dissolved solution and the centrifuged supernatant when cetyltrimethylammonium bromide is added in various concentrations to the concentrated supernatant of broth culture.

**Table 1**

| Endotoxin removing effect of cetyltrimethylammonium bromide | | | | |
|---|---|---|---|---|
| Concentration of cetyltrimethylammonium bromide (mM) | Endotoxin (µg/ml) | | Hemolysin band in SDS-PAGE | |
| | Precipitate | Supernatant | Precipitate | Supernatant |
| 0 | · * | 50.91 | -** | +++*** |
| 1 | 12.78 | 3.83 | +++ | - |
| 2 | 9.08 | 8.79 | +++ | - |
| 5 | 0.74 | 4.71 | +++ | - |
| 10 | 0.04 | 12.82 | +++ | + |
| 20 | 0.009 | 11.98 | ++ | ++ |
| 40 | 0.004 | 8.12 | ++ | ++ |

| | | | | |
|---|---|---|---|---|
| * : No precipitate | | | | |
| ** : No hemolysin band | | | | |
| *** : Density of hemolysin band + < ++ < +++ | | | | |

With the increase of concentration of cetyltrimethylammonium bromide, the quantity of endotoxin in the precipitate-dissolved solution decreased. As a result of SDS-PAGE, hemolysin protein was present only in the precipitate-dissolved solution when final concentration of cetyltrimethylammonium bromide was within the range of 1 to 5 mM. When it exceeds 10 mM, hemolysin protein was found not only in the precipitate-dissolved solution but also in the centrifuged supernatant. Therefore, to effectively remove lipopolysaccharides from hemolysin protein and not to decrease yield of hemolysin protein, it was optimal to set final concentration of cetyltrimethylammonium bromide within the range of 1 to 10 mM.

### Example 2

Lipopolysaccharide removing effect of various chemicals serving as cationic surface active agent was examined. Surface active agents used were lauryltrimethylammonium bromide, tetradecyltrimethyl ammonium bromide, cetyltrimethyl ammonium bromide, lauryl pyridinium chloride and cetylpyridinium chloride. To 5 ml each of concentrated culture supernatant from A. pleuropneumoniae HA-337 strain, 5 ml each of the surface active agents prepared in 2, 10 and 20 mM respectively was added and they were sensitized at room temperature for 30 minutes. They were separated into the supernatant and the precipitate by centrifugation and 10 ml of 1.0 M sodium chloride was added to each precipitate to dissolve it. The quantities of endotoxin in the precipitate-dissolved solution and the centrifuged supernatant were determined by Limulus test and the presence of hemolysin protein was confirmed by SDS-PAGE.

As a result, the lipopolysaccharide removing effect was found in all of the tested cationic surface active agents with final concentration of 1 to 10 mM and hemolysin protein was confirmed to be present in the precipitate (Table 2).

**Table 2**

| Endotoxin removing effect of various cationic surface active agents | | | | | |
|---|---|---|---|---|---|
| Cationic surface active agent | Concentration (mM) | Endotoxin (µg/ml) | | Hemolysin band in SDS-PAGE | |
| | | Precipitate | Supernatant | Precipitate | Supernatant |
| Lauryltrimethylammonium bromide | 1 | 1.94 | 13.37 | +* | + |
| | 5 | 0.83 | 9.50 | +++ | + |
| | 10 | 1.45 | 7.95 | +++ | -** |
| Tetradecyltrimethylammonium bromide | 1 | 4.95 | 28.95 | ++ | +++ |
| | 5 | 0.46 | 15.01 | +++ | - |
| | 10 | 0.012 | 23.11 | ++ | ++ |
| Cetyltrimethylammonium bromide | 1 | 6.52 | 20.59 | +++ | - |
| | 5 | 0.77 | 2.23 | +++ | - |
| | 10 | 0.018 | 23.95 | ++ | ++ |
| Laurylpyridinium chloride | 1 | 2.11 | 26.89 | + | +++ |
| | 5 | 6.65 | 11.94 | +++ | - |
| | 10 | 2.21 | 15.34 | +++ | - |
| Cetylpyridinium chloride | 1 | 13.66 | 23.62 | +++ | + |
| | 5 | 1.42 | 22.40 | +++ | - |
| | 10 | 0.39 | 20.00 | ++ | ++ |

| | | | | | |
|---|---|---|---|---|---|
| * : Density of hemolysin band + < ++ < +++ | | | | | |
| ** : No hemolysin band | | | | | |

### Example 3

In order to confirm whether the lipopolysaccharide removing effect of a cationic surface active agent is also found in other RTX toxins or not, an experiment was carried out on leukotoxin from P. haemolytica and α-hemolysin from E. coli. Used strains were SI-2 strain, which was isolated from field bovine P. haemolytica infection, and S-5 strain of α-hemolysin producing E. coli. After pre-culture of these strains, they were inoculated to 200 ml each of Columia broth supplemented with 0.002% β-NAD and 10 mM calcium chloride and were subjected to shaken culture at 37°C for 4 hours. The culture was centrifuged and the supernatant was concentrated to about 20 ml by ultrafiltration (300 KDa cutoff). To 5 ml each of the concentrated culture supernatant, 5 ml each of cetyltrimethylammonium bromide, prepared in 0, 2, 10 and 20 mM, was added and each of these solutions was sensitized at room temperature for 30 minutes. Each of the solutions was centrifuged into the supernatant and the precipitate and 10 ml of 1.0 M sodium chloride was added to the precipitate to dissolve it. The quantities of endotoxin in the precipitate-dissolved solution and the centrifuged supernatant were determined by Limulus test and the presence of RTX toxin was confirmed by SDS-PAGE, individually.

As the result, lipopolysaccharides bonded to leukotoxin from P. haemolytica and α-hemolysin from E. coli were effectively removed by cetyltrimethyl ammonium bromide with final concentration of 1 to 10 mM (Table 3). It was therefore demonstrated that the lipopolysaccharide removing effect of a cationic surface active agent is found not only in A. pleuropneumoniae but also in other RTX toxin family.

**Table 3**

| Endotoxin removing effect of cetyltrimethylammonium bromide from RTX toxins of P. haemolytica and E. coli | | | | | |
|---|---|---|---|---|---|
| Bacteria (RTX toxin) | Concentration of cetyltrimethyl ammonium bromide (mM) | Endotoxin (µg/ml) | | RTX toxin band in SDS-PAGE | |
| | | Precipitate | Supernatant | Precipitate | Supernatant |
| P. haemolytica (leukotoxin) | 0 | · * | 12.38 | -** | +++*** |
| | 1 | 0.42 | 0.47 | ++ | - |
| | 5 | 0.10 | 0.03 | ++ | - |
| | 10 | 0.08 | 0.01 | ++ | + |
| E. coli (α-hemolysin) | 0 | · | 14.47 | - | +++ |
| | 1 | 0.76 | 0.47 | ++ | - |
| | 5 | 0.003 | 0.53 | + | ++ |
| | 10 | 0.001 | 1.86 | + | ++ |

| | | | | | |
|---|---|---|---|---|---|
| * : No precipitate | | | | | |
| ** : No RTX toxin band | | | | | |
| *** : Density of RTX toxin band + < ++ < +++ | | | | | |

### Experiment 1

Safety of RTX toxin from which lipopolysaccharides were removed by a cationic surface active agent was examined, using target animals. To 400 ml of concentrated culture supernatant from A. pleuropneumoniae HA-337 strain used in Example 1, 1.02 g of cetyltrimethyl ammonium bromide was added and it was sensitized at room temperature for about 30 minutes. Then, it was centrifuged and the precipitate was collected. To this, 100 ml of 1.0 M sodium chloride was added to dissolve the precipitate. Then, 300 ml of distilled water for injection was added to precipitate hemolysin protein again. After centrifugation, it was dissolved again in 1.0 M sodium chloride. By repeating this procedure, lipopolysaccharides in the precipitate could be removed almost completely. Hemolysin thus prepared was adjusted to about 1 mg/ml in protein quantity and 1.0 ml of this was injected intramuscularly in ear root of pig as immunogen. For each test, 3 - 8 pigs with 4 - 7 weeks old were used and lipopolysaccharide-removed hemolysins of different preparation lots were tested by 4 times in all, changing the day of test. As positive control, 1.0 ml of concentrated culture supernatant before processing with cetyltrimethylammonium bromide was injected similarly to two pigs. The response of pigs under test after injection was observed for about 2 hours. After injection, one of control pigs showed depression, hyperpnea and trepidation. On the other hand, among the pigs injected with lipopolysaccharide-removed hemolysin, none showed these symptoms (Table 4) and safety of hemolysin processed with cetyltrimethyl ammonium bromide was demonstrated.

**Table 4**

| Safety test of lipopolysaccharide-removed RTX toxin | | | | |
|---|---|---|---|---|
| Test No. | Injected material | Protein (mg/ml) | Endotoxin (µg/ml) | Number of pigs with symptoms/number of pigs tested |
| 1 | Lipopolysaccharide-removed hemolysin 1 | 1.33 | 0.32 | 0/3 |
| 2 | Lipopolysaccharide-removed hemolysin 2 | 1.40 | 0.36 | 0/8 |
| 3 | Lipopolysaccharide-removed hemolysin 3 | 1.42 | 0.10 | 0/3 |
| 4 | Lipopolysaccharide-removed hemolysin 4 | 1.07 | 0.09 | 0/3 |
| Control | Concentrated culture supernatant without treatment | N D* | 82.88 | 1/2 |

| | | | | |
|---|---|---|---|---|
| * : Not done | | | | |

### Experiment 2

Effectiveness of RTX toxin from which lipopolysaccharides were removed by a cationic surface active agent was evaluated, using experimental small animals. One hundred twenty ddY mice with 3 weeks old (Nippon SLC, Shizuoka, Japan) were divided into 4 groups, each having 30 heads. Lipopolysaccharide-removed hemolysin from A. pleuropneumoniae HA-337 strain used in Experiment 1 was prepared at protein concentration of 120, 12 and 1.2 µg/ml and 0.5 ml each of these immunogens was injected intraperitoneally twice to 30 mice each with an interval of two weeks. As control, 1.0 M sodium chloride which was solvent for hemolysin was injected by the same procedure.

In 20 mice each from each group, 5 mice each were sacrificed by bleeding every week from the first week after the first immunization, and serum was pooled and ELISA antibody titer against hemolysin was determined. The remaining 10 mice of each immunized group, including control, were challenged intraperitoneally with viable A. pleuropneumoniae HA-337 strain of 2.1 x 10⁶CFU/0.5ml at two weeks after the immunization. The mice were observed for one week after the challenge and protective effect was evaluated from survival rate of mice.

Table 5 shows change of serum antibody titer of mice. Antibody titer rapidly increased in the first week after the second immunization depending upon concentration of immunogen. After challenge, all mice in the control group died whereas 90% of mice survived in 60 or 6 µg immune groups, exhibiting high protective effect (Table 6).

**Table 5**

| Change of serum antibody titer | | | | |
|---|---|---|---|---|
| Group | Weeks after first immunization | | | |
| | 1 | 2 | 3 | 4 |
| 60 µg immunization group | <1* | 4 | 256 | 128 |
| 6 µg immunization group | <1 | <1 | 32 | 32 |
| 0.6 µg immunization group | <1 | <1 | 4 | 8 |
| control group | <1 | <1 | <1 | <1 |

| | | | | |
|---|---|---|---|---|
| * : ELISA antibody titer (x 100) against hemolysin | | | | |

**Table 6**

| Protective effect of lipopolysaccaride-removed hemolysin in mice | | | | |
|---|---|---|---|---|
| Challenge dose (CFU/0.5ml/head) | Concentration of immunogen (µg/0.5ml/head) | | | |
| | 60 | 6 | 0.6 | Control |
| 2.1×10⁶ | 9/10* | 9/10 | 5/10 | 0/10 |

| | | | | |
|---|---|---|---|---|
| * : Number of survivals/number tested | | | | |

The above results suggest that lipopolysaccharide-removed hemolysin is useful as vaccine antigen because it increases antibody titer in experimental animal level and has effective protective potency.

According to the present invention, lipopolysaccharides are efficiently removed from RTX toxin, which enables preparation of a vaccine for RTX toxin family having high safety.

## Claims

1. A process for producing a vaccine for bacterial toxin belonging to RTX toxin family which comprises proliferating in a nutrient enriched medium bacteria which produce toxin belonging to RTX toxin family, adding a cationic surface active agent to a concentrated supernatant of a culture to precipitate RTX toxin, not containing lipopolysaccharides, washing the precipitate by distilled water, and using thus produced precipitate as vaccine antigen.

2. A process according to claim 1 wherein concentration of the cationic surface active agent used is within the range of 1 - 10 mM.

## Patentansprüche

1. Verfahren zur Herstellung eines Impfstoffs gegen ein zu der RTX-Toxin-Familie gehörendes bakterielles Toxin, wobei das Verfahren die Schritte Proliferation von Bakterien, die ein zur RTX-Toxin-Familie gehörendes Toxin produzieren, in einem mit Nährstoffen angereicherten Medium, Zugabe eines kationischen Tensids zu einer konzentrierten überstehenden Flüssigkeit einer Kultur zur Präzipitation von RTX-Toxin, die keine Lipopolysaccharide enthält, Waschen des Präzipitats mit destilliertem Wasser und Verwendung des so hergestellten Präzipitats als Vakzinantigen umfasst.

2. Verfahren gemäß Anspruch 1, bei dem die Konzentration des verwendeten kationischen Tensids innerhalb des Bereichs von 1 bis 10 mM liegt.

## Revendications

1. Procédé de production d'un vaccin d'une toxine bactérienne appartenant à la famille des toxines RTX qui consiste à faire proliférer, en un milieu enrichi en substance nutritive, des bactéries qui produisent une toxine appartenant à la famille des toxines RTX, à ajouter un agent de surface cationique à un liquide surnantant concentré d'une culture pour précipiter la toxine RTX, ne contenant pas de lipopolysaccharides, à laver le précipité à l'eau distillée, et à utiliser le précipité ainsi produit comme antigène de vaccin.

2. Procédé selon la revendication 1, dans lequel la concentration de l'agent de surface cationique utilisé est compris dans la plage de 1 à 10 mM.
